# EUROPÄISCHE PATENTSCHRIFT

(11) **EP 2 640 454 B1**
(45) Veröffentlichungstag und Bekanntmachung des Hinweises auf die Patenterteilung: **15.08.2018**
(21) Anmeldenummer: 11819084.2
(22) Anmeldetag: 18.11.2011
(51) Int. Cl.: A61M 27/00

(54) **ELEKTRISCH BETÄTIGBARES, INSBESONDERE PROGRAMMIERBARES HYDROCEPHALUS-VENTIL**
ELECTRICALLY ACTUATABLE, IN PARTICULAR PROGRAMMABLE HYDROCEPHALUS VALVE
VALVE POUR HYDROCÉPHALIE À ACTIONNEMENT ÉLECTRIQUE, NOTAMMENT PROGRAMMABLE

(30) Priorität: 19.11.2010 DE 102010051743
(43) Veröffentlichungstag der Anmeldung: 25.09.2013
(73) Patentinhaber: Christoph Miethke Gmbh & Co. KG, 14469 Potsdam (DE)
(72) Erfinder: MIETHKE, Christoph, 14469 Potsdam (DE)
(74) Vertreter: Kaewert, Klaus
(86) Internationale Anmeldenummer: PCT/EP2011/005819
(87) Internationale Veröffentlichungsnummer: WO 2012/065750

(56) Entgegenhaltungen:
- EP-A1- 1 380 317
- EP-A1- 2 008 683
- DE-U1- 20 121 938

## Beschreibung

An Hydrocephalus erkrankten Menschen haben das Problem, dass ein durch überschüssiges Hirnwasser (Liquor cerebrospinalis) entstehender erhöhter Hirninnendruck zu schwerwiegenden Problemen für die Betroffenen führt. So wird das Hirngewebe geschädigt und dauerhaft abgebaut, es kommt zu unterschiedlichen Symptomen wie Schwindel, Gangstörung, Kopfschmerzen, Übelkeit, Erbrechen und Demenz. Unbehandelt kann die Erkrankung letztendlich zum Tod des Patienten führen. Art und Umfang der auftretenden Beschwerden hängen ab von der Erkrankung zugrundeliegenden Ursachen, allgemeiner Konstitution, vor allem aber auch vom Alter des Patienten. Bei Säuglingen bewirkt der Druckanstieg ein unnatürliches Wachstum des Kopfes, bei Erwachsenen geht die Hirnsubstanz zugunsten des Wasseranteils im Schädelinneren schneller verloren.

Erst seit den fünfziger Jahren des letzten Jahrhunderts steht für Hydrocephaluskranke eine erfolgreiche Behandlungsmöglichkeit zur Verfügung. Es wird eine künstliche Drainage implantiert, die den Abfluss des Hirnwassers in andere Körperregionen ermöglicht, in denen die abgeleitete Flüssigkeit dann abgebaut werden kann. Die Steuerung des Abflusses wird von Ventilen übernommen, die den erforderlichen Druck im Inneren des Kopfes sicherstellen sollen. Seitdem sind eine Vielzahl von verschiedenen technischen Lösungen vorgeschlagen worden, die die Behandlungsmöglichkeiten erweitern oder aber häufig auftretende Komplikationen verhindern oder einschränken sollen.

Es sind bis heute verschiedene Ventilarten am Markt eingeführt worden, die nach ihrem Funktionsprinzip folgendermaßen eingeteilt werden können:

| | fest | einstellbar |
|---|---|---|
| 1. Differenzdruck Ventile | | |
| a: Silikon Schlitz Ventil | √ | × |
| b: Membranventil | √ | × |
| c: Kugel-Konus Ventil | √ | √ |
| 2. Hydrostatische Ventile | | |
| a: Anti-Siphon Ventil | √ | × |
| b: Flusskontrolle | √ | × |
| c: Gravitationsventil | √ | √ |
| √ = verfügbar | x = nicht verfügbar | |

Das starre Differenzdruckventil der Gruppe 1 kann wird als Silikonschlitzventil, als Membranventil oder als Kugel-Konus Konstruktion (US 5069663, DE 30 20 991, US 20100010415) vorgeschlagen. Die Ventile sind in ihrem Öffnungsverhalten auf die liegende Position des Patienten ausgerichtet. In der Stehendposition führen solche Ventile zu systematisch zu Überdrainage, also zu unphysiologisch niedrigen negativem Druck im Kopf des Patienten, was zu schwerwiegenden Komplikationen führen kann. Ventile, die mit einer komplizierten Konstruktion über den Differenzdruck ihre Funktion in der liegenden wie in der stehenden Position erfüllen, haben bislang keinen Weg in die praktische Anwendung gefunden (DE102009009880). Eine grundlegende Verbesserung des Verhaltens von Differenzdruckventilen entsteht durch die Möglichkeit, die Öffnungscharakteristik perkutan einstellen zu können. (US 4772257, EP 0421557A2, US 5928182, EP 135991A1, G8 2143008A, US 4551128, EP 0060369, EP1380317). Eine verbreitete Bauweise (EP1380317) für ein implantierbares Hydrocephalusventil baut auf einem Kugelventil auf, dessen Kugel federbelastet ist. Die Federbelastung der Kugel bestimmt den Öffnungsdruck des Ventils.

Wenn der Liquordruck den Öffnungsdruck des Ventils überschreitet, so öffnet das Ventil und der Liquor strömt aus, bis der Öffnungsdruck wieder unterschritten wird und das Ventil wieder schließt. Die Feder ist eine Blattfeder, die mit dem einen Ende auf der Ventilkugel lastet und mit dem anderen Ende auf einem Rotor aufliegt. Durch Drehung des Rotors wird die Feder verstellt und die Federspannung verändert. Die Verstellung des Rotors erfolgt händisch. Dabei findet eine Verstelleinrichtung außen am Patienten Verwendung. Zur automatischen Verstellung sind Ansätze bekannt, bei denen die Einstellung elektrisch angetrieben und durch Sensorik unterstützt vorgenommen werden kann (EP2008683 von Codman). Durch die Einstellung wird eine individuelle Anpassung der Ventilfunktion an den einzelnen Patienten möglich. Diese Möglichkeit besteht nur für Ventile der Gruppe 1-c, allerdings wird die Abhängigkeit der physikalischen Verhältnisse im Drainagesystem von der Haltung des Patienten auch hier nicht gelöst. Sind die Ventile auf einen niedrigen Öffnungsdruck eingestellt, wird dies zwar einerseits das klinische Ergebnis begünstigen, andererseits wird gleichzeitig die Gefahr der Überdrainage in der Stehendposition dramatisch erhöht. Umgekehrt kann zwar die Einstellung auf einen sehr hohen Wert die Gefahr der Überdrainage reduzieren, gleichzeitig wird dadurch das zu erzielende klinische Ergebnis nachhaltig negativ beeinflusst, da der jetzt anliegende Öffnungsdruck für die Liegendposition deutlich zu hoch ist.

Abhilfe schaffen hier Ventile der zweiten Gruppe. Hydrostatische Ventile zeichnen sich dadurch aus, dass sie die sich durch einen Lagewechsel ändernden physikalischen Bedingungen im Drainagesystem des Patienten berücksichtigen. Drei unterschiedliche Prinzipien werden hierbei genutzt.

Die älteste Konstruktion wurde im sogenannten Antisiphön-Device realisiert. Nach dem gleichen Prinzip sind bis heute mehrere unterschiedliche Konstruktionen am Markt angeboten worden (EP 0670740B1, US 5800376, DE 27 52 087). Hierbei wird die Wirkung des negativen Druckes am Auslass des Ventils systematisch auf ein Minimum reduziert. Diesem Vorteil steht allerdings der gravierende Nachteil gegenüber, dass der Subkutandruck um das Ventilgehäuse einen erheblichen Einfluss auf die Arbeitsweise des Ventils nimmt. Durch Gewebewachstum oder ungünstige Patientenlage kann dieser Druck um erhebliche Werte variieren und dadurch sogar zum absoluten Ventilverschluss führen. Auch diese Ventile konnten die konventionellen Ventile nicht verdrängen(Drake, Toronto).

Das gleiche gilt für das Prinzip der Flusskontrolle. Bei den flussregulierenden Ventilen soll sichergestellt werden, dass die abfließende Menge unabhängig vom am Ventil anliegenden Differenzdruck konstant gehalten wird. Während bei konventionellen Ventilen die Abflussmenge proportional zum anliegenden Differenzdruck steigt, wird dies bei flussregulierenden Ventilen verhindert (Siphonguard [Codman], Orbis Sigma Valve [Cordis], Diamond Valve [Phoenix]; EP 798012A1, US 4627832, US 4776838). Im Mittel beträgt die natürliche Liquorproduktion 23 ml/h. Flussregulierende Systeme haben konkret folgende Probleme:
- Es ist bislang technisch unmöglich, den Wert für die erlaubte Abflussrate sicherzustellen. Varianzen im Rahmen des Produktionsprozesses bleiben zu groß (Aschoff, Schoener).
- Die natürliche Varianz der Produktion bleibt systematisch unberücksichtigt. Liegen die individuellen Werte zu hoch oder zu niedrig, kann dies sowohl zur Überdrainäge als auch zur Unterdrainage führen.
- Die Flussregulierung wird gesteuert über extrem kleine Querschnitte am Öffnungsmechanismus. Partikel im Liquor wie etwa zelluläre Bestandteile nehmen dramatisch Einfluss auf die Funktion und können das Ventil sehr leicht verstopfen. Internationale Vergleichsstudien haben gezeigt, dass dieses Prinzip die Behandlungsergebnisse des Hydrocephalus nicht verbessern konnte (Drake et al).

Die gravitationsunterstützten Ventile werden in zwei Varianten am Markt angeboten. Der erste Ansatz realisiert die Flusssteuerung durch die Schwerkraft gesteuerte Umschaltung von zwei parallel angeordneten Ventilen (DE 4401422, DE 4307387). Die Konstruktion stellt also zwei unterschiedliche Drucksituationen im Ventrikelsystem des Patienten in Abhängigkeit von seiner Haltung ein. Beim zweiten Ansatz wird die Gewichtskraft von Kugeln ausgenutzt, um einen lageabhängig veränderlichen Öffnungsdruck einzustellen (EP 0617975, EP 0115973, DE 19535637). Seit wenigen Jahren existiert ein Gravitationsventil, das über eine perkutan einstellbare Gravitationseinheit verfügt (WO2005092424). Die Technik ermöglicht erstmals individuelle Einstellungen bedingt durch das Körperwachstum bzw. durch die Zunahme des Peritonealdrucks.

Die Aufgabe der Ventrikeldrainage beim Hydrocephalus besteht einerseits in der Ableitung von Hirnwasser zur Verhinderung des pathologischen Druckanstieges, andererseits sollen jedoch gleichermaßen eine ungewollt hohe Drainage und der daraus resultierende extrem negative Druck verhindert werden. Bei allen bisher verfügbaren Ventilen wird dies versucht auf Basis eines Differenzdruckventils. Je nach Bauart sind weitere Einflussfaktoren die Haltung des Patienten, der Subcutandruck oder die Viskosität des Hirnwassers. Der Differenzdruck zwischen Hirnventrikel und Ableitungsmedium (Herzvorhof, freie Bauchhöhle) entscheidet über den Abfluss. Nun können aber sehr unterschiedliche Zustände zu einer erhöhten Druckdifferenz zwischen Hirnventrikel und Ableitungsmedium führen. Es kann sowohl sehr viel Hirnwasser produziert worden sein; es kann aber auch einfach aufgrund des Lagewechsels von der Liegend- in die Stehendposition zu einem erhöhten Druckanstieg gekommen sein. Im ersten Fall muss ein Ventil öffnen, im zweiten Fall sollte dies gerade nicht geschehen. Für ein Differenzdruckventil ist die Situation in beiden Fällen jedoch identisch. Gravitationsventile bieten hier die besten Optionen aber auch hier kann nicht auf temporär veränderte Situationen beispielsweise beim Bauchraumdruck reagiert werden. Klinisch unbefriedigende Befunde können sowohl als Über- als auch als Unterdrainage erklärt werden. Häufig bleibt offen, ob ein optimaler Behandlungserfolg möglich gewesen wäre.

Obwohl durch die beschriebenen Ventilsysteme viele Probleme gelöst werden konnten, bleiben insgesamt folgende Aspekte ungelöst:
1. Eine Anpassung der Ventilcharakteristik an wachstums- oder altersbedingte Veränderungen oder anderweitiger Änderungen von physiologischen Randbedingungen
2. Eine gezielte nicht-invasive Verstellung der Ventileigenschaften mit unterschiedlichen Einstellungen für unterschiedliche Körperlagen des Patienten
3. Eine konsequente Therapierung von Patienten bis hin zur Abstellung der vielleicht überflüssig gewordenen Drainage
4. Die angemessene Anpassung der Liquordrainage an individuelle Besonderheiten
5. Alle bisher angebotenen Lösungen basieren ausschließlich auf dem Differenzdruckprinzip. Andere Parameter, die ebenfalls Einfluss auf die sinnvolle Steuerung bei der Liquordrainage haben könnten, werden nicht erfasst.
6. Die intelligente, situationsabhängig wertende Steuerung von Ventileigenschaften
7. Die nachträgliche Analyse von Vorfällen . In der Regel bleibt die Erklärung der Ursachen für Zwischenfälle bei Vermutungen.

Zur Lösung von verschiedenen Problemen sind Ansätze bekannt gemacht worden, die dies mit Hilfe neuer Ventilverfahren und dafür angepasster Ansteuerungen umsetzen sollen (DE 19915558, DE 19654990, DE 10233601, WO 2010066438).

Zu den bewährten Ventiltechniken, gehören federbelastete Kugelventile, deren Feder verstellbar ist. Zur Verstellung können unterschiedliche mechanische Einrichtungen genutzt werden. Eine besonders beliebte Verstelleinrichtung wird durch einen Rotor gebildet, der in dem Ventil verschwenkbar angeordnet ist. An Umfang ist der Rotor mit einer Gleitfläche versehen, an der die Feder unmittelbar oder mittelbar anliegt, so daß eine Rotorverstellung eine Federverstellung bewirkt (DE102005013720, DE102007059300).

Eine andere bekannte Ventiltechnik benutzt auch einen Rotor zur Federverstellung. Der Rotor besitzt aber keine kurvenförmig verlaufende Mantelfläche, sondern wie Schneckengänge oder wie Gewindegänge verlaufende Flächen, an denen die Feder gleitet. Zumeist ist diese Fläche gestuft (US7235060, US7559912, US2010/0010415).

Die Rotorvorstellung erfolgt zumeist mit Hilfe von Magneten, wobei ein Teil der Magnete im Rotor angeordnet und mit dem Ventil unter der Haut des Patienten implantiert ist und wobei der andere Teil der Magnete auf der Haut des Patienten über dem Rotor gedreht wird. Dadurch bewegen sich die Magnete des Rotors mit dem Rotor.

Es gibt aber auch andere bekannte Vorschläge zur Rotorverstellung, zum Beispiel mit einem Antriebsmotor bzw. mit einem elektromechanischen Aktor. Dabei kann es sich um elektrische Motore , um Magnete und um Linearantriebe handeln (EP2008683)

Der Betrieb der Ventilschalter erfolgt also in bekannten Ausführungen elektromechanisch, elektromagnetisch, in anderer Ausführung aber auch unter Ausnutzung der Eigenschaften von Formgedächtnis Werkstoffen. Dabei ergeben sich Probleme im Energieverbrauch und in der Wärmeentwicklung sowie bei der Biokompatiblität, durch die die Konstruktionen sehr aufwendig werden. Bislang ist nicht bekannt geworden, dass ein programmierbares Ventil am Markt angeboten wird.

Dabei zeigen die bevorzugten Ausführungsformen insbesondere
a)einem mindestens teilweise unabhängig vom Liquordruck betätigbaren Ventil
b)einem elektrisch betätigbaren Aktor für die Ventilbetätigung, vorzugsweise einem piezoelektrischen Aktor (Translator),
c)einem Prozessor und einem Datenspeicher
d)einer Energieeinheit (Batterie)

Vorteilhafterweise sind die erfindungsgemäßen Ausführungsformen auch ohne eine separate Verriegelungsvorrichtung funktionsfähig, wie sie zum Beispiel Gegenstand der EP1380317A1 ist. Die EP1380317 löst ein Problem der aus US7235060, US7559912, US2010/0010415 bekannten Ventile. Dort ist eine Verriegelung der Rotorstellung vor und nach der vorgesehenen Betätigung des Rotors erforderlich, weil vor und nach der Betätigung des Rotors ein Verbleiben des Rotors in der gewählten Stellung nicht gesichert ist. Als Ursache werden vor allem Magnetfelder, insbesondere elektrisch erzeugte Magnetfelder angesehen. Nach dem Vorschlag der EP1380317 wird eine Verriegelung des Rotors mit einem flexiblen Gehäuse erzeugt, das in der Verriegelungsstellung reibungsschlüssig gegen den Rotormantel drückt. Zur Lösung der Verriegelung wird das Gehäuse so verformt, daß sich das Gehäuse seitlich ausbeult und den Rotor für eine Verstellung freigibt. Die Freigabe ist allerdings nicht gleichbedeutend mit einer Verstellung. Für die Verstellung müssen erst die vorstehend erläuterten Magnete aktiviert werden.

Der Prozessor und der Datenspeicher sind vorzugsweise in einer Einheit zusammen gefasst. Im Folgenden wird das als Datenverarbeitungseinheit bezeichnet.

Günstig ist, wenn zugleich ein Druckmesser zur Messung des Liquordruckes und/oder eine Temperaturmesser zur Messung der Liquortemperatur vorgesehen ist, wobei der Druckmesser und/oder Temperaturmesser mit der Datenverarbeitungseinheit gekoppelt ist, so dass die Druckmesssignale in der Datenverarbeitungseinheit aufgenommen und mit einer Vorgabe aus dem Speicher verglichen werden können, um das Ventil solange zu betätigen oder geschlossen zu halten, bis der Liquordruck das vorbestimmte Maß erreicht hat.

Als Druckmesser sind zum Beispiel Einrichtungen geeignet, wie sie beschrieben sind in DD289197A5, DE102006004523A1, DE102006004523A1, DE102005020569B4, DE102004056757A1, DE102004056756A1, DE102004055220A1, DE29705671U1, DE20121938U1, DE20121388U1, DE19713266A1, DE19705474A1, DE19638813C1, DE196009983C1, DE10353144A1, DE10156469B4, DE10156469A1, DE10053409C. Vorzugsweise finden Druckmess-Chips Anwendung, bei denen ein elektrisch leitendes System auf Druckänderungen mit einer Änderung des Widerstandes bei der Stromdurchleitung reagiert.

Dabei kann der Druckmesser so ausgebildet sein, dass die Messsignale in digitaler Form anfallen oder es kann ein Signalwandler zwischen dem Druckmesser und der Datenverarbeitungseinheit vorgesehen sein bzw. an dem Druckmesser oder an der Datenverarbeitungseinheit vorgesehen sein.

Zugleich ist ein Lagedetektor von Vorteil. Auch der Lagedetektor ist dann mit der Datenverarbeitungseinheit gekoppelt, so dass die Signale des Lagedetektors in der Datenverarbeitungseinheit aufgenommen werden können, um den Prozessor von einem Betriebsmodus auf den anderen Betriebsmodus umzuschalten. Dabei ist der eine Betriebsmodus auf die Stehendlage ausgelegt und der andere Betriebsmodus auf die Liegendlage ausgelegt.

Wahlweise ist auch eine Umschaltung auf mindestens einen weiteren zwischenliegenden Betriebsmodus vorgesehen, so dass mindestens ein Übergangsmodus zwischen der Stehendlage und der Liegendlage vorgesehen ist.

Wahlweise kann auch eine gleitende Anpassung an eine Lageänderung stattfinden.

Als Lagedetektor ist zum Beispiel eine Vorrichtung mit einem Gehäuse geeignet, in der eine Kugel je nach Lage des Patienten eine andere Lage einnimmt und in der die Lage der Kugel gemessen wird. Die Lagebestimmung der Kugel kann mit Kontakten erfolgen, welche bei der Berührung mit der Kugel zum Beispiel über den Widerstand unterschiedlichen Einfluss auf die Stromdurchleitung nehmen. Ebenfalls geeignet ist ein Lagesensor, der aus einem Gehäuse mit einer exzentrisch angeordneten Scheibe besteht, die je nach Neigung des Patienten eine andere Lage einnimmt und bei der je nach Lage der Scheibe unterschiedliche, stromdurchflossene Kontakte berührt werden, so dass je nach Lage der Scheibe unterschiedlicher Einfluss auf die Stromdurchleitung genommen wird.

Vorzugsweise findet ein mikro-elektro-mechanisches System zur Lagebestimmung Anwendung, das auf Basis einer Kapazitätsänderung von Kondensatoren arbeitet. Diese Sensoren sind meist aus Silizium hergestellte Feder-Masse-Systeme, bei denen die "Federn" nur wenige µm breite Silizium-Stege sind und auch die Masse aus Silizium hergestellt ist. Durch die Auslenkung bei Beschleunigung kann zwischen dem gefedert aufgehängten Teil und einer festen Bezugselektrode eine Änderung der elektrischen Kapazität gemessen werden. Der gesamte Messbereich entspricht einer Kapazitätsänderung von nur wenige pF, die Änderungen liegen im fF Bereich. Die Elektronik zur Auswertung dieser kleinen Kapazitätsänderung wird auf demselben Halbleiterbaustein integriert. Die Kondensatoren sind in drei Hauptebenen angeordnet, durch die vollständig dreidimensionale Funktionsweise kann jede Körperlage bzw. Lageänderung erfasst werden.

Dabei kann der Lagedetektor so ausgebildet sein, dass die Messsignale in digitaler Form anfallen oder es kann ein Signalwandler zwischen dem Lagedetektor und der Datenverarbeitungseinheit vorgesehen sein bzw. an dem Lagedetektor oder an der Datenverarbeitungseinheit vorgesehen sein.

Ferner können weitere Zustände des Patienten, die Einfluss auf die Liquordrainage bzw. auf die Hydrocephalusbehandlung haben, mit Messeinrichtungen gemessen werden, wobei die Messeinrichtungen mit der Datenverarbeitungseinheit gekoppelt sind, so dass die Signale der Messeinrichtung in der Datenverarbeitungseinheit aufgenommen werden können, um den Prozessor von einem Betriebsmodus auf den anderen Betriebsmodus umzuschalten. Solche Zustände können sich in Abhängigkeit von dem Leiden des Patienten ergeben, das für die Störung des Liquoranfalles oder für die Störung des Liquorflusses oder für die Störung der Liquorresorption ursächlich ist. Solche Zustände können unter anderem Blutdruck und Herzfrequenz sein.

Ferner können bestimmte Anforderungen aus einer körperlichen Betätigung oder einer körperliche Entlastung (Ruhephase) mit in das Programm der Datenverarbeitungseinheit aufgenommen werden.

Wahlweise können verschiedene Messeinrichtungen miteinander kombiniert werden.

Eine EDV-Steuerung für ein Hydrocephalusventil ist bereits aus der DE 10105315 A1 bekannt. Darin ist ein elektromechanisches Ventil beschrieben, das über eine programmierbare EDV-Steuerung betätigt wird. Die EDV-Steuerung soll nach dem Differenzdruck und gegebenenfalls nach der Lage des Patienten sowie nach dem Muskelpotential und Hirnströmen programmiert werden können.

Durch die DE1015315A1 ist auch bekannt, die Einrichtung im Patienten zu implantieren und die gesammelten Daten von außen auszulesen und Programmänderungen von außen vorzunehmen. Das bedingt Sende- und Empfangseinrichtungen.

Die DE10105315A1 schlägt auch eine piezoelektrische Bewegungserzeugung vor.

Die Verwirklichung dieser bekannten Einrichtung mit EDV-Steuerung ist jedoch bislang nicht gelungen.

Zur Verwirklichung dieses bekannten Vorschlages ist nunmehr vorgesehen:
Der Lagesensor/Lagedetektor gibt die Haltung des Patienten an die Steuereinheit weiter. Der von den Drucksensoren/Druckmessgerät gemessene Wert am Ein- und Auslass kann dazu genutzt werden, kurzfristige Änderungen zu erfassen und entsprechend zu berücksichtigen. Sehr schnell ansteigende hohe Druckänderungen entstehen durch einen Lagewechsel, der vom Neigesensor bestätigt werden kann. Im Zusammenhang mit der Kenntnis über die Haltung des Patienten kann der Steueralgorithmus nun angepasst werden. In der stehenden oder sitzenden Position erhöht sich der hydrostatische Druck zwischen dem Ventrikel im Kopf und dem Ableitungsmedium, die Öffnungszeit des Schalters muss sich also verkürzen.

Langsam ansteigende Druckdifferenzen bei gleichbleibender Position des Patienten geben einen Hinweis auf den Hirndruckanstieg. Werden in einem bestimmten Zeitintervall als kritisch vorgegebene Werte erreicht, ohne dass der Patient seine Lage verändert, kann dies als Anstieg des Hirndruckes gewertet werden und die Öffnung des Schalters zur Folge haben. Eine optimale Einstellung der Öffnungszeit lässt sich aus der hydrostatischen Höhe, der Drucksituation an Ein- und Auslass der Drainage sowie der Viskosität der Flüssigkeit errechnen. Die Erfassung des Absolutdruckes vor und hinter dem Schalter liefert Anhaltspunkte für die aktuelle Drainagesituation. Steht beispielsweise bei geschlossenem Schalter ein Patient auf, führt dies sowohl oberhalb als auch unterhalb des Schalters zu charakteristischen Zuständen, die entsprechend kontrolliert werden können.

Sensordaten/Messdaten und Zeitdaten des laufenden Einsatzes können gespeichert werden. Über das Sendemodul können die gespeicherten Daten aus dem Ventilspeicher ausgelesen werden und in die Diagnose einfließen. Die am Schalter gemessene Druckdifferenz kann dabei für die Ermittlung der tatsächlich abgeflossenen Flüssigkeitsmenge genutzt werden, indem sie mit der Öffnungszeit, der Viskosität der Flüssigkeit sowie der Geometrie der Drainage in Beziehung gebracht wird. Mit diesen Daten lässt sich die geflossene Liquormenge in ausreichender Annäherung berechnen.

Die Abspeicherung solcher Daten und das spätere Auslesen zur extrakorporalen Aufbereitung bietet die Möglichkeit, solche Erkenntnisse in die Diagnose und Therapie allgemein sowie bei Zwischenfällen einzubeziehen. Der längerfristige Vergleich ermöglicht Aussagen zum Krankheitsverlauf und zur Erstellung von verbesserten Regelalgorithmen oder Korrekturen. Es können typische, individuell am betroffenen Patienten gewonnene Kurven in den Algorithmus einbezogen werden. Der Vergleich des aktuell gemessenen Geschehens mit gespeicherten typischen Werten kann pathologische Abweichungen aufzeigen, die eine systematische Intervention des Regelalgorithmus nach sich ziehen.

In der Kommunikation zwischen Arzt und Patient können in Abhängigkeit von Beobachtungen, Gewohnheiten und medizinischen Notwendigkeiten optimale Ableitungsprofile erarbeitet, am Implantat eingestellt, systematisch auf ihre Wirksamkeit hin geprüft und gegebenenfalls angepasst werden. Hierdurch kann eine Vielzahl von Revisionen, die nach dem bisherigen Stand der Technik erforderlich wären, vermieden werden.

Es kann von außen durch qualifiziertes Personal eine Programmierung derart vorgenommen werden, dass eine aus den Patientenbedürfnissen abgeleitete Funktion des implantierten Ventils hergestellt werden kann. Es besteht so z.B. die Möglichkeit, eine Anpassung an wachstumsbedingte Änderungen durchzuführen oder Behandlungsprofile für bestimmte spezielle Formen des Hydrocephalus zu programmieren.

Vorteilhafterweise kann der Energiebedarf durch Zu- und Abschalten von Funktionen während des Betriebes verringert werden.

Eine Veringerung des Energiebedarfes ist eine zentrale Frage für Hersteller und Anwender, um eine lange Lebensdauer der implantierten Einheit zu gewährleisten. Zum einen muss dies durch die technische Umsetzung ewährleistet sein, zum anderen soll auch der Arzt die Möglichkeit haben, energieintensive Untersuchungen auf den unmittelbaren Bedarf zu beschränken. Der Arzt soll hierbei einen energiearmen Dauerbetrieb einstellen können und denoch in der Lage sein, kurzfristig intensive Untersuchungen durchzuführen. Hierfür ist ein "Burst Modus" einstellbar, der in hohen Taktraten, vorzugsweise 1 Hz, über einen kurzen Zeitraum von vorzugsweise 10 bis 20 Minuten alle Daten zu Druck, Temperatur, Ventileinstellung und Lage auswertet und an das Steuergerät überträgt.

Bereits in DE 10105315 ist ein programmierbares Hydrocephalus Ventil beschrieben, dass über eine Zeitschaltung der Öffnungszustände einen veränderbaren Abfluss überschüssigen Liquors ermöglicht. Die Ansteuerung des Ventilschalters erfolgt dabei elektromagnetisch durch eine seitliche Verschiebung einer Kugel aus dem Ventilsitz. In DE10233601 wirken zwei in entgegengesetzter Richtung wirkende Formgedächtnis-Drähte als Aktor um eine Kugel seitlich zu verschieben und damit das Ventil zu steuern. In beiden Fällen kommen die Aktoren direkt in Kontakt mit dem Fluid. Sämtliche Materialen und Kontakte müssen daher biokompatibel sein, des Weiteren ist eine elektrische und insbesondere auch thermische Isolierung erforderlich. Trotz der technischen Machbarkeit der beiden oben genannten Prinzipien konnte ein zeitgesteuertes Ventil bis heute nicht zur Marktreife entwickelt werden. Ein langzeitstabile Isolierung und Kapselung der mit dem Liquor in Kontakt stehenden Teile sowie der elektrischen Durchleitungen lässt sich technisch nur schwer realisieren und macht den Aufbau insgesamt zu aufwendig. Der Hauptnachteil im Betrieb ist der hohe Energieverbrauch, der nachteilig auf die Forderung nach einer sehr langen, wartungsfreien Funktionsweise des Implantats wirkt. Die damit verbundene Wärmeentwicklung führt zudem zu Problemen beim Kontakt mit Liquor. Auch die Weiterentwicklung des Formgedächtnis-Antriebs in WO2010066438, bei dem die Drähte nicht in Kontakt mit dem Liquor kommen, behebt diese Probleme nicht.

Die vorliegende Erfindung ermöglicht durch Einführung eines neuartigen, piezoelektrischen angesteuerten Schalters nun erstmals die Realisierung eines elektronisch gesteuerten und elektrisch betriebenen Ventiltyps.

Wesentlicher Bestandteil der Erfindung ist die Ventileinheit bestehend aus einem piezoelektrische Antrieb und einem mechanischem Schaltermechanismus zum Öffnen und Schließen der Drainage. Dieser Öffnungsmechanismus des Ventils besteht aus einem Gehäuse, einem Hebel als Kraft- bzw. Wegübersetzung und einem Ventilsitz, der über den Hebel durch einen Dichtkörper verschlossen wird. Der Hebel fungiert als Kraft- bzw. Weg-Übersetzer-Einheit für einen hier vorzugsweise verwendeten linearen Piezo Aktor. Mit Piezo Aktoren lassen sich hohe Kräfte aber nur vergleichsweise geringe Hübe erzielen, die Stellwege des hier vorzugsweise verwendeten Aktors liegt bei 2 µm bis zu 10 µm. Insbesondere auf die geringen Stellwege wird zurückgeführt, dass der Hinweis in der DE10105315A1 noch keine Umsetzung in der Praxis gefunden hat. Der Verstellweg eines Piezo Aktors ist annäherungsweise linear über die Spannung, d.h. durch die Wahl einer geeigneten Spannung kann eine Anpassung an individuelle Patientenbedürfnisse hergestellt werden. Der mit dem Piezo Aktor erzielbare Hebelweg ist zwar gering, nach der Erfindung wird die mit dem Piezo Aktor verfügbare große Kraft aber genutzt, um durch Übersetzung einen größeren Weg zu schaffen, der für eine Ventilöffnung erforderlich ist. Die Übersetzung wird durch eine Hebelmechanik geschaffen. Die Übersetzung kann in weiten Grenzen nach Belieben gewählt werden. Der Öffnungsspalt kann dadurch unabhängig von der Lage des Patienten kleiner oder größer gestaltet werden. So kann zum Beispiel im Stehen des Patienten ein sehr kleiner Spalt geöffnet werden (Spannung null) und im Liegen des Patienten ein hoher Liquordurchlass eingestellt werden (Spannung maximal). Je nach medizinischer Vorgabe können auch andere Spalte eingestellt werden. Die Steuerung des Öffnungsspaltes zur Regulierung der geringen Hydrocephalus-typischen Flussraten ist damit ohne weiteres möglich. Die Übersetzung wird vorzugsweise durch eine Hebelmechanik bewirkt. Die Hebelmechanik wird wahlweise durch einen zweiarmigen Hebel gebildet. Zu dem Hebel gehört eine Hebellagerung.

Durch die Übersetzung, die mit einem Verhältnis von vorzugsweise ca. 1:2 bis 1:10 realisiert werden kann, wird die Öffnungsweite des Ventils konstruktiv bestimmt.

Die Hebellagerung kann durch ein herkömmliches Schwenklager gebildet werden. Vorzugsweise wird die Hebellagerung aber durch eine elastische Membran gebildet. Die Membran kann durch ein separates Bauteil gebildet werden. Die Membran kann aber auch mit dem Hebel einteilig ausgebildet sein.

Diese Membran kann zumindest folgende Funktionen: Sie kann sowohl dienen
1. als Lagerung des Hebels,
2. als Rückstellfeder, die den unbelasteten Hebel in seine eindeutige Ausgangsposition bringt sowie
3. als Abdichtung des Ventilinnenraumes gegenüber dem Außenraum.

Die Hebelachse liegt also in einer lagerbildenden Membran, die eine kinematische Fixierung der Drehachse erzwingt und damit ein elastisches Lager für den Hebelmechanismus bildet. Bei doppelarmigem Hebel ist der eine Hebelarm an der einen Membranseite angeordnet und der andere Hebelarm an der anderen Membranseite. Vorzugsweise befindet sich der Piezo Aktor an dem kürzen Hebelarm und wirkt der längere Hebelarm auf das Ventil. Das wirksame Verhältnis der Hebelarme bestimmt das Übersetzungsverhältnis. Die Öffnungsweite des Ventils ist dann bestimmt durch die Auslenkung des längeren Teils des Hebels, sie ist um das Hebelverhältnis/Übersetzungsverhältnis größer als die Auslenkung des Piezo Aktors.

Das Ventil kann verschieden gestaltet werden.

Zum Beispiel kann es sich um ein Kugelventil oder um ein Membranventil oder um ein Klappenventil oder um ein Konusventil handeln.

Vorzugsweise ist ein Ventil mit einer Kugel-Konus Paarung vorgesehen. Diese Variante hat sich für Hydrocephalus Ventile bewährt und wird in zahlreichen existierenden Produkten eingesetzt (DE 19535637). Prinzipiell kann der Sitz auch anders gestaltet sein, beispielsweise nadelförmig, kegelförmig oder planar.

Die Kugel ist im Ventilsitz geführt und vorzugsweise nicht mit dem Hebel verbunden. Sobald das Ventil über den der Hebel geöffnet wird, erzeugt der einlassseitige Überdruck des Liquors einen entsprechenden Spalt in der Kugel-Konus Paarung und die Flüssigkeit kann fließen. Die Rückstellung erfolgt nach Abschalten des Piezo Aktors automatisch durch die elastische Membran. In der Ausgangsstellung liegt die Kugel vorzugsweise unter Vorspannung in dem Ventilsitz. Dazu ist der Hebel so ausgebildet, dass die Kugel zurück in den konischen Ventilsitz gepresst wird. Die Vorspannung des Hebels in Nullstellung, also bei geschlossenem Ventil, dient dazu, einen dichten Sitz der Kugel auch bei Erschütterungen sicherzustellen.

Vorzugsweise besitzt das Ventil zwei durch die Membran hermetisch getrennte Räume. Der eine Raum ist der Ventilinnenraum, im obigen Fall der Raum mit Kugel-Konus. Der Raum wird bei geöffnetem Ventil von Liquor durchströmt. Der andere Raum wird als Bauraum bezeichnet. Vorzugsweise befinden sich in dem Raum
die elektrischen Bauteile,
die Batterie und
der Piezo Aktor.

Der durch den Ventilinnenraum strömende Liquor wird über den Auslass in den Bauchraum abgeleitet.

Der Bauraum kapselt die darin befindlichen Teile ab, so dass der Bauraum auch dann eine Biokompatibilität der eingeschlossenen Teile bewirkt, wenn diese Teile ohne Kapselung keine Biokompatibilität besitzen.

Die Kapselung verhindert auch ein Eindringen von Flüssigkeit in den Bauraum und die Berührung von Flüssigkeit mit dem Piezo Aktor.

Alle elektrischen Bauteile befinden sich vorzugsweise im Bauraum. Die Isolierung von elektrischen Leitern zwischen Ventilinnenraum und Bauraum kann dann entfallen. Die Kapselung bewirkt damit euch eine elektrische Isolierung und verhindert, dass durch die erforderlichen Spitzenspannungen von bis zu 150 Volt eine Gefahr für den Patienten entsteht. Die lagerbildende Membran wird vorzugsweise mit dem inneren Ventilgehäuse verschweißt. Das bewirkt eine form- und stoffschlüssige Verbindung. Die Membran ist nach dem Verschweißen wie auch nach anderen Verbindungen raumtrennend mit dem Gehäuse dicht verbundenSie bildet das Schwenklager für den Hebel.

Für Anwendungsfälle mit anderen Rahmenbedingungen als die hier beschriebenen oder die Verwendung eines anderen Aktors mit größerem Verstellweg können die Hebellängen a:b grundsätzlich in drei Abhängigkeiten gewählt werden:
- a<b
- a=b
- a>b

Vorteilhafterweise ist das erfindungsgemäße Hebellager bzw. deren Membran mit der räumliche Trennung(Medientrennung) zwischen Elektronik und durchfließendem Medium nicht nur bei einer Übersetzung, sondern auch bei einer Übersetzung von 1:1 oder auch bei einer Untersetzung der Hebel-Antriebsbewegung anwendbar.

Die Öffnung des Ventils ist grundsätzlich auch mit anderen Antrieben möglich, beispielsweise über einen Hubmagneten oder über einen Motor. So können beispielsweise Antriebe gestaltet werden durch:
- Piezo-Aktor (Stack, Biegewandler, etc.)
- DC-Motor, Schritt-, Servo-, Torquemotor
- Hubmagnet
- MEMS, Memoryantriebe
- Magnetostriktiver Aktor

Die vorzugsweise Verwendung eines Piezo Aktors bietet den Vorteil, dass nur ein sehr geringer Energiebedarf entsteht. Im Normalbetrieb muss der Piezo zudem nicht bis zur vollen Auslenkung angesteuert werden, wodurch der Energiebedarf weiter gesenkt wird. Das Halten einer Auslenkung erfordert nur noch sehr geringeEnergie. Hierdurch wird die Lebensdauer des Implantats deutlich erhöht.

Entsprechend dem geringen Energiebedarf ist auch die mögliche Umwandung von von Energie in Wärme gering.

Um die Elektronik zu schützen wird die Elektronik ganz oder teilweise vergossen z.B. in ein Polyurethan oder Epoxydharz. Bei Deformationen des Gehäuses können so ein Kurzschluss oder andere Gefahren für den Patienten verhindert werden.

Vorzugsweise wird die Batterie mitimplantiert.

DieBatterie verfügt je nach Wahl über eine Spannung von 2,8 bis 3,2 Volt. Zum Einsatz kommt vorzugsweise eine Lithium-Jod Batterie mit 2,8 Volt, die heute üblicherweise für Herzschrittmachen eingesetzt werden. Derartige Batterien werden beispielsweise von der Firma Greatbatch Medical angeboten.

Bei höherer Arbeitsspannung des Piezo Aktors erfolgt vorzugweise eine Umspannung der Batteriespannung auf die Arbeitsspannung.

In der Zeichnung ist ein Ausführungsbeispiel der Erfindung dargestellt.

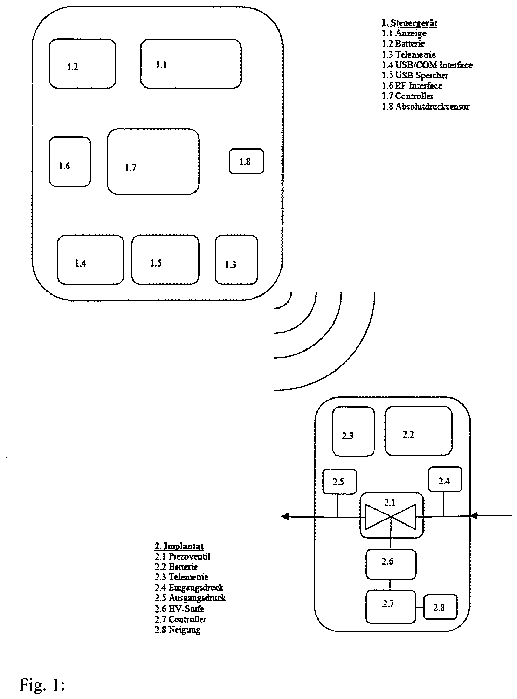

Fig. 1a und 1b zeigen ein extrakorporal angeordnetes Steuergerät(Steuerung CPU) 1.0 und ein Implantat 2.0 in einem Funktionsschaubild mit einer Auflistung der wesentlichen Komponenten. In Fig. 1b ist zusätzlich ein externer Computer als Konfigurationsgerät 3.0 dargesetllt.

Das Steuergerät 1.0 besteht aus einer Anzeige 1.1, einer Batterie 1.2, einer Sende- und Empfangseinheit(telemetrischer Modul) 1.3, einer USB Com Interface 1.4, ein USB speicher 1.5, einem RF Interface, einem Controller 1.7 und einem Absolutdrucksensor 1.8.

Das Implantat 2.0 besteht aus einem piezobetätigten Kugelventil 2.1, einer Batterie 2.2, einer Sende- und Empfangseinheit 2.3, einem Messgerät für den Eingangsdruck2.4, einem Messgerät für den Ausgangsdruck 2.5, einer HV-Stufe, einem Controller 2.7 und einem Neigungsmesser 2.8.

Die Batterie 2.2 kann eine induktiv aufladbare Batterie.

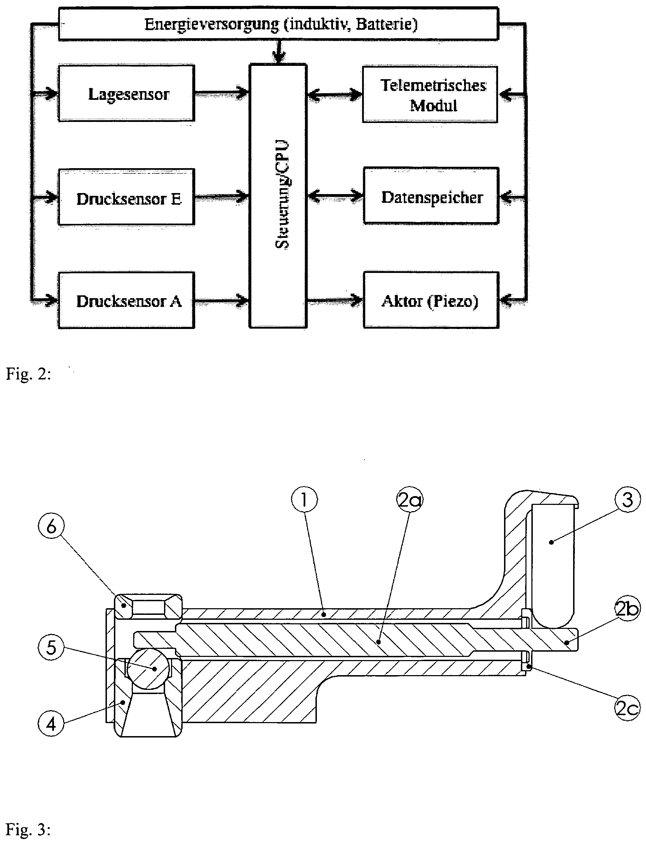

Fig. 2 zeigt ein Blockschaltbild zur Verdeutlichung des Zusammenwirkens von Steuergerät 1.0 und Implantat 2.0. Daraus ergibt sich, dass das Steuergerät 1.0 mit den Messgeräten des Implantats und dem Aktor des Piezoventils im Implantat kommuniziert, wobei sowohl auf gespeicherte Daten Rückgriff genommen wird, wie auch neue Daten gespeichert werden.

Neben der Programmierung findet eine Synchronisation von Daten und Uhrzeit durch das Steuergerät statt. Abgefragt wird die ID des Implantats, der Batteriestatus und die Betriesbsart. Die Uhrzeit wird korrigiert/synchronisiert. Es werden dabei alle weltweiten Zeitzonen unterstützt. Ein wesentlicher Austausch findet laufend zur Qualität der telementrischen Ankopplung statt. Daten und Energie hierzu werden visualisieret.

Am Eingabegerät 3.0, das ein Computer, ein Smartphone oder ein ähnliches, mobiles Gerät sein kann, wird einerseits das Implantat grundkonfiguriert. Andererseits werden die vom Lesegerät übertragenen Konfigurationen eingestellt. Dies kann insbesondere sein;
die Vorgabe der Ventilzustände für einen Zeitabschnitt, vorzugsweise 24 Stunden, die Parameter zur individuellen Anpassung der lagebedingten Öffnungscharakteristik sowie
die Betriebsart (Datenaufzeichnung, Ventilsteuermodus, Schnelldatenerfassung).

Fig. 3 zeigt Einzelheiten des Piezo-Ventils. Der Einlass 4 besitzt einen kegelförmigen Ventilsitz, in den eine Ventilkugel 5 eingepasst ist und dem gegenüber der Auslass 6 liegt. Das Ventilkammergehäuse 1 weist eine durchgehende Bohrung auf, durch die ein Hebelarm 2a bis zur Kugel ragt. Die Hebelarm 2a lastet mit seinem einen Ende auf der Ventilkugel 5, er wird vom elastischen Lager 2c gehalten. Der über das Ventilgehäuse 1 hinausragende Hebelarm 2b ist um 90Grad abewinkelt. Das Verringert das Bauvolumen des Ventils. Je kleiner das Ventil ist, desto mehr Tragekomfort ergibt sich. Der Hebelarm 2b wird mittels eines Piezo Aktors 3 bewegt. Zu dem Schwenkhebel 2b gehört ein Schwenklager. Hier wird das Schwenklager durch eine Membran 2c gebildet. Die Membran 2c bewirkt zugleich eine Trennung von Ventilinnenraumraum und Außenraum. Die Membran bildet außerdem eine Feder. Durch die Federkraft wird der Hebel in seine Ausgangsstellung zurückgeführt.Die Membran und der Hebel werden zumindest teilweise auf einer Drehbank gefertigt. Deer abgewinkelte Hebelarb 2b wird gefräst.. Der Hebel und die Membran besitzen einen kreisförmigen Querschnitt, welche die Fertigung auf der Drehbank erlauben. Die Membran besteht im Ausführungsbeispiel aus Titan mit einer Dicke von 0,18 mm und ist mit der Ventilkammer verschweißt.

In anderen Ausführungsbeispielen besitzt die lagerbildende Membran eine ovale Form.

In noch anderen Ausführungsbeispielen besitzt der Hebel einen eckigen, vorzugsweise quaderförmigen Querschnitt und eine quaderförmige Membran.

Diese Bauformen bieten Möglichkeiten zur Anpassung an vorgegebene geometrische Gegebenheiten, die Form des Ventilgehäuses.

Bei der dargestellten Ausführungsform ist der Hebel in allen Richtungen quer zur Längsrichtung mit gleicher Kraft verformbar. Um die Bewegung in nur einer Richtung sicher zu stellen kann dem Lager ein Verstärkung parallel zur gewünschten Schwenkachse des Hebels angeformt werden. Zum Beispiel versteift eine Wulst die lagerbildende Membran so, dass sich die gewünschte Biegung einstellt. Die Wulst verläuft in einer Ebene, auf der die Antriebsbewegung des Piezo-Aktors senkrecht steht.

Ähnliche Wirkung wie ein Wulst kann auch von einer Nut ausgehen, die in die Membran eingearbeitet werden kann.

Ähnliche Wirkung kann auch mit einer Profilierung der Membran erzeugt werden. So kann die Membran auch wellenförmig gestaltet werden, um ein möglichst weiches Federverhalten zu erzielen. Dies kann in Fällen, in denen es auf einen exakten Ventilsitz ankommt. Die axiale Nachgiebigkeit und damit das Verrücken der Lagerachse während der Stellbewegung wird wahlweise bewusst in Kauf genommen.

In anderen Ausführungsbeispielen ist ein anderes biokompatibles Material als Titan, gegebenenfalls auch Kunststoff, vorgesehen. Bewegt sich der Hebel, so dass die Kugel 5 entlastet wird, wird die Kugel durch einen mit Überdruck anfallenden Liquor aus dem Ventilsitz bewegt, das Ventil öffnet. Ein evtl. auftretender Gegendruck auf der Auslassseite bei geöffnetem Schalter führt automatisch zum Schließen des Ventils durch die vom Medium in den Kegelsitz 4 gedrückte Kugel, so dass es nicht zu einem Rückfluss kommen kann.

Um das Ventil unabhängig vom Gegendruck wieder zu schließen, muss nur die an dem Piezo Aktor anliegende Spannung abfallen. Die Kugel 5 wird wieder in den Kegelsitz 4 gegen die Einlassöffnung gedrückt. Dies wird primär durch die Membran 2c und sekundär durch den als Stabfeder wirkenden Hebel 2a bewirkt, die zusammen das Federsystem bilden.

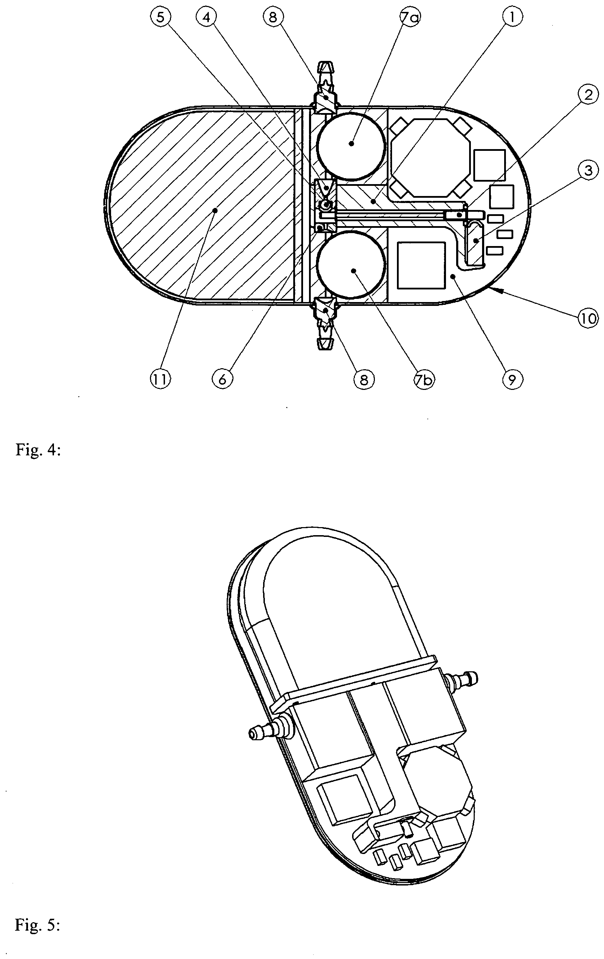

Fig. 4 und 5 zeigen (einmal in einer Explosionsdarstellung, einmal in einer perspektivischen Teilansicht) den Gesamtaufbau eines solchen Ventils in technischer Ansicht.

Dabei wird zwischen zwei Räumen beiderseits der Membran 2c in Fig. 3 liegen.

Der eine Raum gehört zu dem Strömungskanal des Liquor durch das Ventil und grenzt im Ausführungsbeispiel an die Membranseite, welche der Kugel 5 zugewandt ist.

Der andere Raum grenzt an die gegenüberliegende Membranseite und wird hier als Bauraum bezeichnet.

Der Bauraum wird begrenzt durch das Gehäuse 10, das wie die anderen metallischen Teile vorzugsweise aus Titan, vorzugsweise TiAl6V4, besteht.

Die linke Hälfte des Bauraumes wird durch eine Batterie 11 ausgefüllt. Die notwendige Energie für die Betätigung des Piezo Aktors liefert vorzugsweise eine kleinvolumige Knopfbatterie. Auch bei geringen Batteriespannungen von zum Beispiel 3V lässt sich eine piezoelektrische Federverstellung mit Betriebsspannungen bis zu 150 V betreiben. Die Batteriespannung wird dazu entsprechend transformiert.

Mittig, oben und unten am Gehäuse 10 befinden sich die Ein- und Auslasstüllen 8, die zu dem Strömungskanal des Liquor durch das Ventil gehören. In dem Strömungskanal befinden sich zwei Drucksensoren/Druckmesser mit integriertem Temperaturmesser 7a und 7b. In der Mitte der beiden Sensoren/Druckmessern befindet sich die in Fig. 1 beschriebene Ventilkammer 1, wiederum vorzugsweise aus Titan gefertigt. An der rechten Seite befindet sich der Piezo-Aktor 3. Die restlichen Bauteile sind Controller für den Programmablauf, ein Lagesensor und ein Spannungsumwandler, um aus der Batteriespannung die nötige Spannung für den Piezo-Aktor bereitzustellen. Diese Bauteile sind in dem verbliebenen Raum 9 angeordnet.

Eine am Piezo Aktor 3 angelegte Spannung bewirkt eine Verschiebung, die den Hebel 2 an seinem kürzeren Ende 2b auslenkt. Die Auslenkung des längeren Hebalarmes bewirkt eine Öffnung des Ventils wobei die Öffnungsweite abhängig ist von der Höhe der angelegten Spannung und der Hebelübersetzung oder Hebeluntersetzung. Die Bohrung des Kegelsitzes 4 weist typischerweise einen Durchmesser von etwa 1 mm bis 2 mm auf, vorzugsweise 1,5 mm, bei einem Kugeldurchmesser von typischerweise 1,6 bis 2,5 mm. Bei geringer Liquorviskosität kann auch ein Konusdurchmesser von 0,5 mm zielführend sein, wobei dann der Kugeldurchmesser zwischen 0,7 und 1,5 mm liegen kann. Die Kugel 5 besteht vorzugsweise aus einem harten und leichten Material, beispielsweise Aluminiumoxidkeramik. Sie hat einen etwas größeren, hier vorzugsweise einen etwa 1,3-fach größeren Durchmesser als die Durchlassbohrung des Kegelsitzes 4.

In Fig. 4 ist auf der Untersesite dse Gehäues ein speziell bearbeiteter Bereich vorgesehen. Dieser liegt gegenüber der die Platine mit der Spule zur induktien Energieversorung und telemetrischen Datenübertragung. Zur Minimierung der Dämpfung des Signals ist die Gehäusewand an dieser Stelle verdünnt. In der dargestellten Ausführung ist die Gehäusewand durch Materialabtrag bearbeitet worden, wobei einige Stege zur Stabilisierung stehen gelassen worden sind. In anderen Ausführungsbeispielen wird die Gehäusewand durch eine eingeschweißte Folie gebildet. Hierzu eignen sich Folien mit einer Dicke von 0,012m bis 0,1m Dicke.

## Patentansprüche

1. Implantierbares Hydrocephalusventil (2.0; 2.1; 2.2; 2.3; 2.4; 2,5; 2,7; 2.8) mit einer elektrischen Betätigung, mit einer EDV-Steuerung (1.0; 1.1; 1.2; 1.3; 1.4; 1.5; 1.7; 1.8), die in Wirkverbindung mit mindestens einem Druckmesser, vorzugsweise auch in Wirkverbindung mit einem Lagedetektor steht, und die Druckmesswerte und die Neigungsmesswerte mit einer Vorgabe vergleicht, um bei wesentlicher Abweichung das Ventil zu betätigen,
wobei durch das Ventil ein Strömungskanal für den Liquor führt und wobei die Schließeinrichtungen des Ventils in dem Strömungskanal angeordnet sind **dadurch gekennzeichnet, dass** die Betätigungseinrichtung für das Ventil aus einer Hebelmechanik (2; 2a, 2b) und einem Antrieb (3) besteht, wobei mindestens eine Hebellagerung (2c) durch eine Membran gebildet wird, die mindestens den Antrieb (3) gegenüber dem Liquor kapselt.

2. Vorrichtung nach Anspruch 1, **dadurch gekennzeichnet, daß** die Membran (2c) fest auf dem Hebel (2a) sitzt.

3. Vorrichtung nach Anspruch 1, **dadurch gekennzeichnet, daß** die Membran (2c) mit dem Hebel (2a) einstückig ist.

4. Vorrichtung nach einem der Ansprüche 1 bis 3, **dadurch gekennzeichnet, dass** der Strömungskanal für den Liquor sich bis zu einer Seite der Membran (2c) erstreckt und dass sich der gegenüber dem Liquor gekapselte Raum (9) bis zur anderen Seite der Membran (2c) erstreckt.

5. Vorrichtung nach einem der Ansprüche 1 bis 4, **dadurch gekennzeichnet, daß** die Membran (2c) die Hebellagerung bildet.

6. Vorrichtung nach einem der Ansprüche 1 bis 5, **dadurch gekennzeichnet, dass** die Hebelmechanik (2;ein Übersetzungsgetriebe ist.

7. Vorrichtung nach Anspruch 6, **gekennzeichnet durch** eine 2fache bis 10fache Übersetzung der Antriebsbewegung.

8. Hydrocephalusventil nach einem der Ansprüche 1 bis 7, **dadurch gekennzeichnet, dass** der Antrieb (3) durch einen Piezo-Aktor gebildet wird.

9. Vorrichtung nach einem der Ansprüche 1 bis 8, **dadurch gekennzeichnet, dass** der Antrieb (3) aus der Gruppe der DC-Motore, Schrittmotore, Servomotore, Torquemotore ausgewählt ist.

10. Vorrichtung nach einem der Ansprüche 1 bis 7, **dadurch gekennzeichnet, dass** der Antrieb (3) durch einen Hubmagneten gebildet wird.

11. Vorrichtung nach einem der Ansprüche 1 bis 7, **dadurch gekennzeichnet, dass** der Antrieb (3) aus der Gruppe der MEMS, Memoryantriebe ausgewählt ist.

12. Vorrichtung nach einem der Ansprüche 1 bis 7, **dadurch gekennzeichnet, dass** der Antrieb (3) als magnetostriktiver Aktor ausgebildet ist.

13. Vorrichtung nach einem der Ansprüche 1 bis 12, **gekennzeichnet durch** einen elektrischen Antrieb (3) mit einer Batterie, vorzugsweise einer induktiv aufladbaren Batterie.

14. Vorrichtung nach Anspruch 13, **gekennzeichnet durch** eine zwischen Batterie und Antrieb (3) oder an dem Antrieb (3) vorgesehenen Umspannvorrichtung zur Erhöhung der Batteriespannung auf die Spannung des Antriebes (3).

15. Vorrichtung nach einem der Ansprüche 1 bis 14, **gekennzeichnet durch** Anordnung mindestens einen Druckmessers in Strömungsrichtung des Liquors vor und hinter dem Ventil oder im Ventil.

16. Vorrichtung nach Anspruch 15, **gekennzeichnet durch** die Verwendung von Druckmessern mit einem digitalen Messsignal oder einem nachgeschalteten Wandler für das Messsignal.

17. Vorrichtung nach einem der Ansprüche 1 bis 16, **gekennzeichnet durch** Anordnung mindestens eines Lagedetektors mit einem digitalen Messsignal oder einem nachgeschalteten Wandler für das Messsignal vor oder hinter dem Ventil oder im Ventil.

18. Vorrichtung nach einem der Ansprüche 1 bis 17, **gekennzeichnet durch** die Anordnung mindestens eines Temperaturmesser (7a; 7b) mit einem digitalen Messsignal oder einem nachgeschalteten Wandler für das Messsignal vor oder hinter dem Ventil oder im Ventil.

19. Vorrichtung nach einem der Ansprüche 1 bis 18, **gekennzeichnet durch** einen Prozessor und Datenspeicher (1.5) im Implantat für die Ventilsteuerung und mit einer Datenverbindung zwischen dem Ventil und den Messvorrichtungen

20. Vorrichtung nach einem der Ansprüche 1 bis 19, **gekennzeichnet durch** ein Implantat mit einer Datenverbindung zwischen dem Ventil und den Messvorichtungen sowie einer telemetrischen Messwertübertrag an ein außen liegendes Steuerungsgerät, das mit Prozessor und Datenspeicher versehen ist.

21. Vorrichtung nach Anspruch 19 oder 20, **gekennzeichnet durch** einen programmierbaren Prozessor

22. Vorrichtung nach einem der Ansprüche 1 bis 21, **gekennzeichnet durch** einen runden, vorzugsweise einen kreisförmigen Querschnitt des Hebels (2) .

23. Vorrichtung nach einem der Ansprüche 1 bis 21, **gekennzeichnet durch** einen eckigen Querschnitt des Hebels (2), vorzugsweise einen quaderförmigen Querschnitt des Hebels (2).

24. Vorrichtung nach Anspruch 22, **gekennzeichnet durch** eine - in der Ansicht entlang dem Hebel - runde, vorzugsweise eine kreisförmige lagerbildende Membran (2c).

25. Hydrocephalusventil nach Anspruch 23, **gekennzeichnet durch** eine - in der Ansicht entlang dem Hebel - eckige, vorzugsweise quaderförmige lagerbildende Membran(2c) .

## Claims

1. Implantable hydrocephalus valve with an electrically operated valve (2.0; 2.1; 2.2; 2.3; 2.4; 2.5; 2.7; 2.8), with a computer control (1.0; 1.1; 1.2; 1.3; 1.4; 1.5; 1.7; 1.8) that operatively connects with at least one pressure gauge, preferably also operatively connected with a position detector, and compares the pressure gauge values and the inclination values with a pre-setting in order to actuate the valve in the event of a significant deviation,
wherein a flow channel for the liquor leads through the valve and wherein the closing devices of the valve are positioned in the flow channel
**characterised in that** the actuation device for the valve consists of a lever mechanism (2; 2a, 2b) and a drive mechanism (3), wherein at least one lever bearing (2c) is formed by a membrane that encapsulates at least the drive mechanism (3) against the liquor.

2. Device according to claim 1, **characterised in that** the membrane (2c) is firmly seated on the lever (2a).

3. Device according to claim 1, **characterised in that** the membrane (2c) is integrally formed with the lever (2a).

4. Device according to one of claims 1 to 3, **characterised in that** the flow channel for the liquor extends to one side of the membrane (2c) and that the space (9) that is encapsulated against the liquor extends to the other side of the membrane (2c).

5. Device according to one of claims 1 to 4, **characterised in that** the membrane (2c) forms the lever bearing.

6. Device according to one of claims 1 to 5, **characterised in that** the lever mechanism (2) is a step-up gear unit.

7. Device according to claim 6, **characterised by** a 2x to 10x step up of the drive movement.

8. Hydrocephalus valve according to one of claims 1 to 7, **characterised in that** the drive (3) is formed by a piezo actuator.

9. Device according to one of claims 1 to 8, **characterised in that** the drive (3) is selected from the group of the DC motors, step motors, servo motors, torque motors.

10. Device according to one of claims 1 to 7, **characterised in that** the drive (3) is formed by a solenoid.

11. Device according to one of claims 1 to 7, **characterised in that** the drive (3) is selected from the group of the MEMS, memory drives.

12. Device according to one of claims 1 to 7, **characterised in that** the drive (3) is formed as a magnetostrictive actuator.

13. Device according to one of claims 1 to 12, **characterised by** an electrical drive (3) with a battery, preferably an inductively chargeable battery.

14. Device according to claim 13, **characterised by** a transformer device provided between battery and drive (3) or on the device provided on the drive (3) for increasing the battery voltage to the voltage of the drive (3).

15. Device according to one of claims 1 to 14, **characterised by** locating at least one pressure gauge in the flow direction of the liquor before and after the valve or in the valve.

16. Device according to claim 15, **characterised by** the use of pressure gauges with a digital measurement signal or a downstream converter for the measurement signal.

17. Device according to one of claims 1 to 16, **characterised by** locating at least one position detector with a digital measurement signal or a downstream converter for the measurement signal before or after the valve or in the valve.

18. Device according to one of claims 1 to 17, **characterised by** locating at least one temperature measurement device (7a; 7b) with a digital measurement signal or a downstream converter for the measurement signal before or after the valve or in the valve.

19. Device according to one of claims 1 to 18, **characterised by** a processor and data memory device (1.5) in the implant for the valve control and with a data link between the valve and the measurement devices.

20. Device according to one of claims 1 to 19, **characterised by** an implant with a data link between the valve and the measurement devices as well as a telemetric measurement value transmission to an external control apparatus that is equipped with processor and data memory device.

21. Device according to claim 19 or 20, **characterised by** a programmable processor.

22. Device according to one of the claims 1 to 21, **characterised by** a round, preferably a circular cross section of the lever (2).

23. Device according to one of the claims 1 to 21, **characterised by** a square cross section of the lever (2), preferably a rectangular cross section of the lever (2).

24. Device according to claim 22, **characterised by** a - in the view along the lever - round, preferably a circular bearing-forming membrane (2c).

25. Hydrocephalus valve according to claim 23, **characterised by** a - in the view along the lever - square, preferably a rectangular bearing-forming membrane (2c).

## Revendications

1. Valve implantable pour hydrocéphalie (2.0, 2.1, 2,2, 2.3, 2.4, 2.5, 2.7, 2.8) avec un actionnement électrique, avec une commande informatique (1.0, 1.1, 1.2, 1.3, 1.4, 1.5, 1.7, 1.8) qui est en liaison active avec au moins un capteur de pression, de préférence également en liaison active avec un capteur de position, et qui compare les mesures de pression et les valeurs mesurées d'inclinaison à une consigne, afin d'actionner la valve en cas d'écart significatif, un conduit d'écoulement pour le liquide céphalorachidien passant à travers la valve et les dispositifs de fermeture de la valve étant disposés dans le conduit d'écoulement, **caractérisée en ce que** le dispositif d'actionnement pour la valve se compose d'un mécanisme de levier (2, 2a, 2b) et d'un entraînement (3), au moins un palier de levier (2c) étant formé par une membrane qui encapsule au moins l'entraînement (3) par rapport au liquide céphalorachidien.

2. Dispositif selon la revendication 1, **caractérisé en ce que** la membrane (2c) est fixée sur le levier (2a).

3. Dispositif selon la revendication 1, **caractérisé en ce que** la membrane (2c) est d'un seul tenant avec le levier (2a).

4. Dispositif selon l'une des revendications 1 à 3, **caractérisé en ce que** le conduit d'écoulement pour le liquide céphalorachidien s'étend jusqu'à un côté de la membrane (2c) et **en ce que** l'espace (9) encapsulé par rapport au liquide céphalorachidien s'étend jusqu'à l'autre côté de la membrane (2c).

5. Dispositif selon l'une des revendications 1 à 4, **caractérisé en ce que** la membrane (2c) forme le palier de levier.

6. Dispositif selon l'une des revendications 1 à 5, **caractérisé en ce que** le mécanisme de levier (2) est un engrenage de transmission.

7. Dispositif selon la revendication 6, **caractérisé par** une transmission de 2 à 10 fois du mouvement d'engrenage.

8. Valve pour hydrocéphalie selon l'une des revendications 1 à 7, **caractérisée en ce que** l'entraînement (3) est formé par un actionneur piézo-électrique.

9. Dispositif selon l'une des revendications 1 à 8, **caractérisé en ce que** l'entraînement (3) est choisi à partir du groupe de moteurs DC, de moteurs pas à pas, de servomoteurs, de moteurs couple..

10. Dispositif selon l'une des revendications 1 à 7, **caractérisé en ce que** l'entraînement (3) est formé d'un aimant de levage.

11. Dispositif selon l'une des revendications 1 à 7, **caractérisé en ce que** l'entraînement (3) est choisi à partir du groupe de MEMS, entraînements à mémoire.

12. Dispositif selon l'une des revendications 1 à 7, **caractérisé en ce que** l'entraînement (3) est réalisé en tant qu'actionneur magnétostrictif.

13. Dispositif selon l'une des revendications 1 à 12, **caractérisé par** un entraînement (3) électrique avec une batterie, de préférence une batterie rechargeable inductivement.

14. Dispositif selon la revendication 13, **caractérisé par** un dispositif de transformation prévu entre la batterie et l'entraînement (3) ou au niveau de l'entraînement (3) pour élever la tension de la batterie à la tension de l'entraînement (3).

15. Dispositif selon l'une des revendications 1 à 14, **caractérisé par** l'installation au moins d'un capteur de pression dans le sens d'écoulement du liquide céphalorachidien avant et après la valve ou dans la valve.

16. Dispositif selon la revendication 15, **caractérisé par** l''utilisation de capteurs de pression avec un signal de mesure numérique ou un convertisseur en aval pour le signal de mesure.

17. Dispositif selon l'une des revendications 1 à 16, **caractérisé par** l'installation au moins d'un capteur de position avec un signal de mesure numérique ou un convertisseur en aval pour le signal de mesure avant ou après la valve ou dans la valve.

18. Dispositif selon l'une des revendications 1 à 17, **caractérisé par** l'installation au moins d'un capteur de température (7a, 7b) avec un signal de mesure numérique ou un convertisseur en aval pour le signal de mesure avant ou après la valve et dans la valve.

19. Dispositif selon l'une des revendications 1 à 18, **caractérisé par** un processeur et un stockage de données (1.5) dans l'implant pour la commande de la valve et avec une liaison des données entre la valve et les dispositifs de mesure.

20. Dispositif selon l'une des revendications 1 à 19, **caractérisé par** un implant avec une liaison des données entre la valve et les dispositifs de mesure ainsi qu'une transmission des valeurs de mesure télémétrique au niveau d'un appareil de commande placé à l'extérieur qui est muni d'un processeur et d'un stockage des données.

21. Dispositif selon la revendication 19 ou 20, **caractérisé par** un processeur programmable.

22. Dispositif selon l'une des revendications 1 à 21, **caractérisé par** une section transversale arrondie, de préférence circulaire, du levier (2).

23. Dispositif selon l'une des revendications 1 à 21, **caractérisé par** une section transversale angulaire du levier (2), de préférence une section transversale de forme quadrangulaire du levier (2).

24. Dispositif selon la revendication 22, **caractérisé par** une membrane (2c), en vue le long du levier, ronde, de préférence circulaire, formant palier.

25. Valve pour hydrocéphalie selon la revendication 23, **caractérisée par** une membrane (2c), en vue le long du levier, carrée, de préférence de forme parallélépipédique, formant palier.
